# EUROPEAN PATENT SPECIFICATION

(11) **EP 3 563 719 B1**
(45) Date of publication and mention of the grant of the patent: **08.04.2020**
(21) Application number: 18020185.7
(22) Date of filing: 01.05.2018
(51) Int. Cl.: A45D 34/04

(54) **COSMETIC COMPOSITIONS**
KOSMETISCHE ZUSAMMENSETZUNGEN
COMPOSITIONS COSMÉTIQUES

(43) Date of publication of application: 06.11.2019
(73) Proprietor: The Boots Company PLC, Nottingham NG90 1BS (GB)
(72) Inventor: Billinger, Emma, Wollaton, Nottingham NG8 2QU (GB); Fowler, David, Beeston, Nottingham NG9 5NX (GB); Lewis, Melanie, Long Eaton, Nottingham NG10 3DF (GB)
(74) Representative: Agashi, Kapil

(56) References cited:
- WO-A2-2011/073898
- FR-A1- 3 002 123
- FR-A1- 3 009 781
- US-A1- 2015 053 224
- US-A1- 2016 296 436
- US-A1- 2017 164 717

## Description

### FIELD OF THE INVENTION

The present invention relates to a container for packaging and dispensing a cosmetic make-up composition on keratin materials, said container allowing a controlled distribution of a fluid makeup composition.

### BACKGROUND TO THE INVENTION

Consumers are increasingly seeking individuality in their cosmetics, and this in turn has led to a recent increase in ways of obtaining personalized cosmetics. Consumers are, for example, increasingly using beauty smartphone apps that suggest products that suit them.

Concentrated pigment drops provide a form of personalized cosmetics. Consumers choose pigment drops that best suit their skin tone and then add these to their skin care composition of choice (such as moisturiser, serum or face oil), thus obtaining a combination that provides perfect coverage and the correct balance of skin actives. This is of particular appeal to consumers with sensitive skin who previously, once finding a cosmetics range that did not result in skin irritation, were reluctant to change cosmetics and so felt limited in terms of their look. Pigment drops allow such consumers to convert their trusted skin care composition into a foundation or concealer. A further layer of personalization is provided with these drops in that consumers can choose how many drops to apply to their skin care composition, with some pigment drop products currently on the market stating, for example, that a "sheer" level of coverage is obtained with one drop, a "medium" level of coverage is obtained with two drops and a "full" level of coverage is obtained with three drops.

Consumers can also add the pigment drops to colour cosmetics, such as a foundation, a concealer, a tinted moisturiser, a BB cream, a CC cream or a DD cream, as well as the skin care formulations discussed above. Consumers may wish to do this if they cannot find a colour cosmetic product that perfectly suits their skin tone, or for consumers that wish to add coverage to a colour cosmetic for special occasions (such as for a party or evening event) or for dealing with undesirable skin appearance (such as acne blemishes).

Furthermore, pigment drops can be used in isolation (i.e. applied directly to the skin of the consumer) or as a concealer (a cosmetic used to conceal imperfections on the face, such as dark spots, acne scars or discolouration).

With such pigment drop formulations it is important that a high level of droplet reproducibility is provided; consumers want to feel confident that a one, two, three, four or five-drop application will be consistent day after day. If a smaller than normal drop forms upon application then the consumer will have to either accept a level of coverage that is less than they are used to or add a further drop with the risk that they may have more coverage than normal. If a larger than normal drop forms upon application then the consumer will have to either accept a level of coverage that is higher than they are used to or discard some of the drop formation (which can be costly if the pigment drops are expensive). The same is true if the pigment drop formulation is used in isolation; the consumer using a pigment drop formulation with a high level of droplet reproducibility will become accustomed to the coverage by a single droplet and be able to use the formulation with ease each day to achieve the same look. This consumer would understandably get frustrated if the droplet size varies greatly from day to day and as a result, for example, pigment drop formulation is wasted as a result of the droplet size being too large on some days.

Some pigment drop products in the art are provided in a dropper-bottle with a pipette. Whilst such dropper bottles can be effective for formulations with a low viscosity, when used in combination with high viscosity formulations, the formulation has a tendency to stick to the outside of the pipette when the pipette is pulling out of the bottle. The formulation on the outside of the pipette can then move to the end of the pipette during use contribute to the droplet being expelled from the inside of the pipette. This can in turn lead to increased droplet variability. Such dropper-bottle packaging can also be messier during use, as the formulation present on the outside of the pipette can then run down the outside of the bottle as the pipette is being placed back within the bottle.

US2016/0296436 relates to a packaging and application device of a cosmetic composition comprising (1) a container containing said cosmetic composition, the container being a flexible-walled tube; and (2) a dispensing head comprising a dispensing orifice, the area having the smallest cross-section in respect of the flow of the composition between the container and the dispensing orifice is between 0.2 mm² and 3 mm². No mention regarding the viscosity of the cosmetic composition is made therein.

There remains a need for a means of providing consistent droplets of high-pigment formulations.

### SUMMARY OF THE INVENTION

The inventors have surprisingly demonstrated that, through formulating a high pigment formulation in such a manner that the viscosity of the formulation (measured at 23 °C) is 10,000 centipoise (cps, equivalent to 10 Pascal seconds (Pa.s)) or above results in an improvement in droplet reproducibility when dispensed through a flexible-walled container with a dispensing orifice that is between 0.2 mm² and 2.0 mm² in smallest cross-sectional area of passage. This is contrary to previous teachings that droplet reproducibility is achieved through developing formulations that have a low viscosity. Examples of such low-viscosity formulations that have a high level of droplet reproducibility known in the art include eye drops.

Thus, in one aspect the present invention provides a container comprising a flexible wall and a dispensing head, wherein (1) the dispensing head is not detachable from the rest of the container during normal use; (2) the dispensing head comprises a dispensing orifice; (3) the smallest cross-sectional area of passage through the dispensing orifice is between 0.2 mm² and 2.0 mm² and (4) the container contains a fluid skin colour cosmetic composition having a viscosity of at least 10,000 cps measured at 23 °C and comprises at least 20% of one or more pigments.

Alternatively viewed, the present invention provides a container comprising a flexible wall and a dispensing head, wherein (i) the dispensing head is not detachable from the rest of the container during normal use; (ii) the dispensing head comprises a dispensing orifice; (iii) the smallest cross-sectional area of passage through the dispensing orifice is between 0.2 mm² and 2.0 mm² and (iv) the container contains a fluid skin colour cosmetic composition comprising at least 20% of one or more pigments, characterised in that the fluid skin colour cosmetic composition has a viscosity of at least 10,000 cps (10 Pa.s) measured at 23 °C.

In one embodiment the fluid skin cosmetic composition has a viscosity of less than 70,000 cps (70 Pa.s) measured at 23 °C. In one embodiment the fluid skin cosmetic composition has a viscosity of at least 20,000 cps (20 Pa.s), preferably at least 22,000 cps (23 Pa.s), more preferably at least 23,000 cps (23 Pa.s), more preferably at least 24,000 cps (24 Pa.s), more preferably at least 25,000 cps (25 Pa.s), measured at 23 °C.

In one embodiment the fluid skin cosmetic composition comprises no more than 40% of one or more pigments. In one embodiment the fluid skin cosmetic composition comprises at least 22% of one or more pigments, preferably at least 24% of one or more pigments, more preferably at least 26% of one or more pigments, more preferably at least 27% of one or more pigments.

In one embodiment the container is a bottle, preferably a tottle.

In one embodiment the smallest cross-sectional area of passage through the dispensing orifice is between 0.3 mm² and 2.0 mm², preferably between 0.3 mm² and 1.6 mm², more preferably between 0.4 mm² and 1.2 mm², more preferably between 0.6 mm² and 1.0 mm².

In one embodiment the one or more pigments present in the fluid skin cosmetic composition comprise inorganic pigments. In a further embodiment, the one or more pigments comprise titanium dioxides, iron oxides, ultramarines, chromium oxides, chromium hydroxides or mixtures thereof.

In one embodiment the fluid skin cosmetic composition is a pigment drop composition, a concealer, a foundation, a bronzer, a BB cream or a CC cream. Preferably the fluid skin cosmetic composition is a pigment drop composition.

In a further aspect, the present invention provides a method of converting a skincare composition into a colour cosmetic composition comprising dispensing one to five drop(s) from the container of the present invention onto an amount of skincare composition suitable for application to one or more of the skin regions selected from the list consisting of (a) one or more specific parts of the face; (b) the face as a whole; (c) the neck; and (d) the décolletage.

In a further aspect, the present invention provides a method of altering the colour of a colour cosmetic composition comprising dispensing one to five drop(s) from the container of the present invention onto an amount of colour cosmetic composition suitable for application to one or more of the skin regions selected from the list consisting of (a) one or more specific parts of the face; (b) the face as a whole; (c) the neck; and (d) the décolletage.

In a further aspect, the present invention provides a method of converting a skincare composition into a colour cosmetic composition or of altering the colour of a colour cosmetic composition comprising dispensing one to five drop(s) from the container of the present invention onto an amount of the skincare composition or of the colour cosmetic composition suitable for application to one or more of the skin regions selected from the list consisting of (a) one or more specific parts of the face; (b) the face as a whole; (c) the neck; and (d) the décolletage.

### DETAILED DESCRIPTION OF THE INVENTION

### Applicator

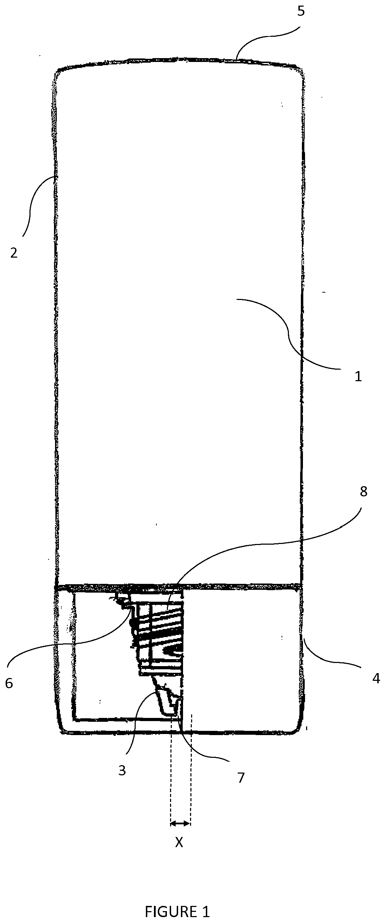

The container 1 comprises a flexible wall 2 and a dispensing head 3 closing the container 1. An example of the container is shown in longitudinal section in Figure 1. Preferably the container 1 is a tottle as shown in Figure 1. The term "tottle" is understood to mean a bottle that can sit on the cap 4 end. Tottles are often used as containers for compositions with a high viscosity, such as ketchup and conditioner, as it allows the consumer to stand the container on the cap 4 when not in use and, in doing so, allows the composition contained therein to move to the dispensing head. This then allows the composition to be dispensed straightforwardly when the container is next used. Preferably the bottom 5 of the container, that is to say the end opposite the dispensing head 3, is curved so that the container 1 cannot be stood upright on the bottom, in order to prevent the composition therein to accumulate away from the dispensing head 3.

The container 1 contains a fluid skin colour cosmetic composition (not shown). The wall 2 is deformable to the touch so that the distribution of the composition is generated by a pressure of the consumer on the wall.

The bottom 5 of the container is closed in a leaktight manner, for example by welding the flexible wall 2.

The dispensing head 3 is not detachable from the rest of the container during normal use. In other words, the base 6 of the dispensing head 3 is fixed to the container 1 by any known means, in particular by welding or overmolding, overmolding being applicable to tube containers in particular. In a variant, with respect to tube containers, the dispensing head 3 can be made integral with the wall 2 of the container. Thus, the container 1 is not, for example, in the form of a dropper bottle where the dispensing head is detachable from the rest of the container during normal use.

The container 1 is for example made of a thermoplastic material. Preferably, the container 1 is made of a multilayer material. With respect to bottle and tottle containers, the material may comprise a polyethylene layer that provides a good level of structural integrity (strong enough to hold the composition and stand upright, but also deformable at the wall for dispensing), for example a blend of high density polyethylene (HDPE) and low density polyethylene (LDPE) (such as 40% HDPE/ 60% LDPE). Preferably, in addition to the polyethylene layer, the bottle or tottle can also comprise an inner ethylene vinyl alcohol (EVOH) layer that prevents the composition from reacting with the polyethylene layer.

With respect to tube containers, the multilayer material may comprise a layer of metallic material, for example made of aluminum, arranged between two layers of thermoplastic material such as polyolefins such as polypropylene or polyethylene. Adhesive layers may be inserted between each of the thermoplastic and/or metal layers. The thickness of each layer may be between 10 µm and 400 µm.

The container made of a multilayer material has the advantage of limiting exchanges between the product contained in the container and the outside. This is particularly useful when the composition contained therein comprises volatile compounds.

In a further embodiment, the dispensing head 3 may be made of a thermoplastic material that is different from that of the rest of the container 1. The dispensing head 3 is for example made of a thermoplastic material such as polypropylene or polyethylene.

The dispensing head 3 has a dispensing orifice 7. The dispensing head 3 is advantageously a cannula extending in the axis of the container, at least the external surface of which preferably converging towards the dispensing orifice 7. The smallest cross-sectional area of passage through the dispensing orifice (X) is between 0.2 mm² and 2.0 mm². Preferably the smallest cross-sectional area of passage through the dispensing orifice is between 0.3 mm² and 2.0 mm², preferably between 0.3 mm² and 1.6 mm², more preferably between 0.4 mm² and 1.2 mm², more preferably between 0.6 mm² and 1.0 mm².

The dispensing head 3 may include a thread 8 so as to receive a cap 4 in order to seal the container 1 by screwing onto the thread 8 of the dispensing head 3. The cap 4 may include a protuberance to be inserted into the dispensing orifice 7 in the screwed-on position on the container so as to close off said orifice 7.

In a preferred embodiment, the container 1 may contain a glass or metal ball (not shown) to aid the mixing of compositions contained therein before use. This can be particularly useful with respect to compositions that are prone to separating when still.

### Fluid skin colour cosmetic composition

The term "fluid" is understood to mean, according to the invention, a composition which flows under its own weight at ambient temperature, as opposed to a "solid" composition. Preferably the colour cosmetic composition has a viscosity of less than 80,000 centipoise (cps, equivalent to 80 Pascal seconds (Pa.s)). More preferably the cosmetic composition has a viscosity of less than 70,000 cps (70 Pa.s).
More preferably the cosmetic composition has a viscosity of less than 60,000 cps (60 Pa.s). More preferably the cosmetic composition has a viscosity of less than 50,000 cps (50 Pa.s). More preferably the cosmetic composition has a viscosity of less than 40,000 cps (40 Pa.s). More preferably the cosmetic composition has a viscosity of less than 30,000 cps (30 Pa.s). In all of the embodiments discussed above, viscosity is measured at 23 °C.

The cosmetic composition present within the container of the present invention has a viscosity of at least 10,000 cps (10 Pa.s). Preferably the cosmetic composition present within the container of the present invention has a viscosity of at least 12,000 cps (12Pa.s). More preferably the cosmetic composition present within the container of the present invention has a viscosity of at least 14,000 cps (14 Pa.s). More preferably the cosmetic composition present within the container of the present invention has a viscosity of at least 16,000 cps (16 Pa.s). More preferably the cosmetic composition present within the container of the present invention has a viscosity of at least 18,000 cps (18 Pa.s). More preferably the cosmetic composition present within the container of the present invention has a viscosity of at least 20,000 cps (20 Pa.s). More preferably the cosmetic composition present within the container of the present invention has a viscosity of at least 22,000 cps (22 Pa.s). More preferably the cosmetic composition present within the container of the present invention has a viscosity of at least 23,000 cps (23 Pa.s). More preferably the cosmetic composition present within the container of the present invention has a viscosity of at least 24,000 cps (24 Pa.s). More preferably the cosmetic composition present within the container of the present invention has a viscosity of at least 25,000 cps (25 Pa.s). In all of the embodiments discussed above, viscosity is measured at 23 °C.

Viscosity is a measure of its resistance to gradual deformation by shear stress or tensile stress, where a liquid with a greater viscosity has a greater resistance to gradual deformation (and in an informal sense is "thicker") than a liquid with a lesser viscosity. The skilled person would be well aware of how to determine the viscosity of a given liquid. A suitable method for measuring viscosity is presented in Example 1.

The skilled person would appreciate that it is very difficult to predict droplet reproducibility based on the viscosity of a liquid and the orifice size, and so, when developing a pigment drop composition would instead look to examples in the art where droplet reproducibility is achieved. The skilled person would, prior to this application, therefore predict that liquids with a low viscosity, such as eye drops, provide a high level of droplet reproducibility, whilst liquids with a high viscosity, such as ketchup, provide a low level of droplet reproducibility (in this regard the skilled person would be aware that, with the example of ketchup in a flexible wall container, a high level of force needs to be applied to the wall of the bottle in order to push the liquid out, and it is common for this force to result in either no ketchup being dispensed or an undesirably high amount of ketchup being dispensed). This is why, in the case of the high-pigment formulations of the present invention, it is particularly surprising that a high level of viscosity, together with a relatively smaller orifice size, is required in order to produce droplet reproducibility.

The term "skin" is understood to mean a composition suitable for application to the skin or to the lips, in particular the skin of the face, neck and décolletage.

The term "colour cosmetic" is understood to mean a composition comprising pigments, which are pulverulent substances intended to bring a color and an aesthetic effect to the skin to which it is applied. Preferably the colour cosmetic composition present within the container of the present invention will be a cosmetic composition for making up the skin of the face, such as a pigment drop or a concealer.

The colour cosmetic composition present within the container of the present invention comprises at least 20% of one or more pigments. In one embodiment, the fluid skin cosmetic composition comprises at least 22% of one or more pigments, preferably at least 24% of one or more pigments, more preferably at least 26% of one or more pigments, more preferably at least 27% of one or more pigments. In one embodiment, the fluid skin cosmetic composition comprises no more than 40% of one or more pigments.

By "pigments", it is necessary to include white or colored, mineral or organic particles (nacre type), insoluble in an aqueous solution, intended to color and/or to opacify the resulting film. Mention may be made, as inorganic pigments which can be used in the invention, of titanium, zirconium or cerium oxides, as well as oxides of zinc, iron or chromium, ferric blue, manganese violet, ultramarine blue and chromium hydrate. Preferably, the composition of the invention comprises at least titanium oxides and iron oxides.

According to one particular embodiment, the composition of the invention comprises at least coated iron oxides and/or coated titanium oxides, preferably coated with aluminum stearoyl glutamate or perfluoroalkyl phosphate.

According to another particular embodiment, the composition present in the container of the present invention comprises at least titanium oxides, preferably titanium dioxide sold under the name Flamenco Summit Gold Y30D by BASF Personal Care Ingredients (INCI name: Mica (and) titanium dioxide) and/or titanium dioxide coated with perfluoroalkyl phosphate, in particular sold under the name PF 5 TiO₂ A 100 by Daito Kasei Kogyo (INCI name: titanium dioxide (and) C₉ to C₁₅ fluoroalcohol phosphate) and coated or uncoated iron oxides.

The term "nacres" is understood to mean colored particles of any shape, iridescent or otherwise, in particular produced by certain shells in their shells or else synthesized and which exhibit an effect of color by optical interference.

Examples of nacres include nacreous pigments such as titanium mica coated with an iron oxide, mica coated with bismuth oxychloride, titanium mica coated with chromium oxide, nacreous pigments based on bismuth oxychloride. They may also be mica particles on the surface of which are superimposed at least two successive layers of metallic oxides and / or organic coloring matter.

According to a particular embodiment, the dyestuffs are inorganic pigments chosen from titanium oxides, iron oxides and mixtures thereof.

Minerals, such as talc or mica, as well as boron nitride, may be used as pigments in the context of the present invention. Synthetic equivalents to these minerals can also be used. For example fluorphlogopite, a synthetic material very similar to mica, may be used.

Polymers can be used to form pigments of the present invention. Polymers may be silicone or non-silicone based. Non-silicone based polymers include nylon, polyamides such as polyhexamethylene adipamide (PA66), polycaproamide (PA6), PA6.10, PA10.10 and PA12, polyesters, polyolefins, polymers based on a cellulose ester, such as cellulose acetate, cellulose propionate, rayon, viscose and polymers of the same family, acrylic polymers, such as poly(methyl methacrylate), and copolymers, copolymers in any proportions of these polymers, and blends between any of these polymers. Preferably the polymer is nylon, such as nylon 6/12, nylon 66, nylon 6, nylon 510 or nylon 1,6 (preferably nylon 6/12). Silica (or a combination of a polymer of silica) may also be used to form the pigment.

Examples of Sensient polymer include COVABEAD LH 85 (methyl methacrylate cross polymer with a matte effect), COVABEAD LH 170 (methyl methacrylate cross polymer with a creamy feeling), COVABEAD PMMA (polymethyl methacrylate with a powdery feeling), COVABEAD VELVET 10 (polymethyl methacrylate with a superior softness effect), COVABEAD VELVET 20 (polymethyl methacrylate with a superior ball-bearing effect) and COVABEAD PMMA 2 MUSI (polymethyl methacrylate with silica).

Suitable Shin Etsu silicone-based polymer include KMP-590 (polymethylsilsesquioxane at an average diameter of 2 µm), KMP-591 (polymethylsilsesquioxane at an average diameter of 5 µm), KSP-100 (vinyl dimethicone/ methicone silsesquioxane crosspolymer at an average diameter of 5 µm), KSP-101 (vinyl dimethicone/ methicone silsesquioxane crosspolymer at an average diameter of 12 µm), KSP-102 (vinyl dimethicone/ methicone silsesquioxane crosspolymer at an average diameter of 30 µm), KSP-105 (vinyl dimethicone/ methicone silsesquioxane crosspolymer at an average diameter of 2 µm), KSP-300 (diphenyl dimethicone/vinyl diphenyl dimethicone/ silsesquioxane crosspolymer), KSP-411 (polysilicone-1 crosspolymer) and KSP-441 (polysilicone-22).

Borosilicates can be used to form pigment. Suitable borosilicate platelets include violet interference pearl. Suitable borosilicate particles include HOLLOW CORE SILICATE R3178 from Sensient, a calcium aluminium borosilicate.

Glass can be used to form pigment. Suitable glass particles include COVABEAD CRYSTAL from Sensient, which are transparent spherical beads.

According to a particular embodiment, the pigments are coated. Preferably this coating is with triethoxycaprylylsilane.

A composition of the invention may be a dispersion or an emulsion.

A dispersion may be carried out in the aqueous phase or in the oily phase.

The compositions of the invention may be in the form of an oily solution or dispersion, a gel, an oil-in-water (O/W), W/O or multiple emulsion, or an anhydrous fluid, in particular an anhydrous fluid. An emulsion may have an oily or aqueous continuous phase. Preferably the composition is a W/O emulsion.

The oil phase may be made up partially or entirely of silicones. Emulsions where the oil phase is made up entirely of silicones may be described as water-in-silicone or silicone-in-water emulsions. Preferably the composition is a water-in-silicone emulsion.

According to a first embodiment, this is an emulsion. In another embodiment, it is anhydrous.

Such an emulsion may be, for example, an inverse (W/O) or direct (O/W) emulsion, or a multiple emulsion (W/O/W or O/W/O). In the case of emulsions, the direct emulsions (O/W) are preferential.

### Aqueous phase

The composition present in the container of the present invention may comprise an aqueous phase.

The aqueous phase comprises water. A water suitable for the invention may be a floral water such as blueberry water and/or mineral water such as VITTEL water, LUCAS water or LA ROCHE POSAY water and/or thermal water.

The aqueous phase may also comprise water-miscible organic solvents (at room temperature, 23 °C), for example monoalcohols containing from 2 to 6 carbon atoms, such as ethanol or isopropanol; the polyols having in particular from 2 to 20 carbon atoms, preferably having from 2 to 10 carbon atoms, and preferably having from 2 to 6 carbon atoms, such as glycerol, propylene glycol, butylene glycol, pentylene glycol , hexylene glycol, dipropylene glycol, diethylene glycol; glycol ethers (in particular having from 3 to 16 carbon atoms) such as mono-, di- or tripropylene glycol (C₁ to C₄) alkyl ether, mono-, di- or triethylene glycol (C₁ to C₄) alkyl ethers , and mixtures thereof.

The aqueous phase may further comprise stabilizing agents, for example sodium chloride, magnesium dichloride and magnesium sulfate.

The aqueous phase may also comprise any water-soluble or water-dispersible compound compatible with an aqueous phase, such as gelling agents, film-forming polymers, thickeners, surfactants and mixtures thereof.

In particular, a composition of the invention may comprise an aqueous phase in a content varying from 1% to 70% by weight, in particular from 5% to 50%, and more particularly from 10% to 45% by weight relative to the weight total composition.

According to one embodiment, the cosmetic make-up composition comprises less than 20% water, preferably less than 10% water, preferably less than 5% water, or even less than 2% water, or even preferably less than 1% of water.

The compositions of the invention may comprise one or more monoalcohol(s) containing from 2 to 8 carbon atoms, in particular from 2 to 6 carbon atoms, and in particular from 2 to 4 carbon atoms. This monoalcohol can be represented, for example, by the formula RₐOH, in which Rₐ represents a linear or branched alkyl group containing from 2 to 8 carbon atoms. Mention may be made, as monoalcohol, of ethanol, isopropanol, propanol or butanol. According to one embodiment, the compositions of the invention comprise ethanol.

The amount of monoalcohol(s) may range from 5% to 40% by weight in the composition, preferably from 10% to 20% by weight and even more preferably from 10% to 15% by weight relative to the total weight of said composition.

According to one particular embodiment, the make-up cosmetic comprises one or more monoalcohols comprising from 2 to 8 carbon atoms, in a content which may range from 5 to 20% by weight, in particular from 10 to 15% by weight per relative to the total weight of the composition.

### Fatty phase

The cosmetic composition present within the container of the present invention may comprise at least one liquid fatty phase. In particular, a composition of the invention may comprise at least one liquid fatty phase, in particular at least one oil, as mentioned below.

Oil is understood to mean any fatty substance in liquid form at ambient temperature (between 20 °C and 25 °C) and at atmospheric pressure. These oils can be of animal, vegetable, mineral or synthetic origin.

According to one embodiment, the fatty phase of the compositions of the invention comprises at least one volatile oil and/or at least one non-volatile oil.

According to a particular and preferred embodiment, the cosmetic make-up composition comprises at least one fatty phase comprising volatile or non-volatile, silicone or hydrocarbon-based oils, and preferably a mixture of volatile and non-volatile silicone and hydrocarbon oils.

### Volatile oils

According to one embodiment, the fatty phase of the composition present within the container of the present invention comprises at least one volatile oil. The fatty phase of the compositions of the invention may comprise a mixture of several volatile oils.

The term "volatile oil" is understood to mean any nonaqueous medium capable of evaporating from the skin or the lips, in less than one hour, at ambient temperature and atmospheric pressure. The volatile oil is a volatile cosmetic oil, liquid at ambient temperature. More specifically, a volatile oil has an evaporation rate of between 0.01 and 200 mg/cm²/min, inclusive.

To measure this rate of evaporation, a crystallizer, 7 cm in diameter, placed on a scale in a large enclosure of about 0.3 m³, temperature controlled, is introduced at a temperature of 25 °C, at a relative humidity of 50%, 15 g of oil or of mixture of test oil. The liquid is allowed to evaporate freely, without agitation, by providing ventilation by a fan (PAPST-MOTOREN, reference 8550 N, rotating at 2700 rpm) placed vertically above the crystallizer containing said oil or said mixture, the blades being directed towards the crystallizer and at a distance of 20 cm from the bottom of the crystallizer. The mass of oil remaining in the crystallizer is measured at regular intervals. The evaporation rates are expressed in mg of evaporated oil per unit area (cm²) and per unit time (minute).

The volatile oils may be hydrocarbon, silicone or fluorinated.

For the purposes of the present invention, "silicone oil" is understood to mean an oil comprising at least one silicon atom, and in particular at least one Si-O group.

The term "fluorinated oil" means an oil comprising at least one fluorine atom. The term "hydrocarbon oil" is understood to mean an oil containing mainly hydrogen and carbon atoms.

The oils may optionally comprise oxygen, nitrogen, sulfur and/or phosphorus atoms, for example, in the form of hydroxyl or acid radicals.

The volatile oils may be chosen from hydrocarbon-based oils containing from 8 to 16 carbon atoms, and especially branched alkanes, in particular C₈ to C₁₆, in particular C₈ to C₂₀, (also called isoparaffins or isoalkanes), such as isododecane (also called 2,2,4,4,6-pentamethyl), isodecane isohexadecane isoeicosane and, for example, the oils sold under the trade names ISOPARS® or Permethyls®.

As volatile hydrocarbon-based oils, mention may also be made of linear C₉-C₁₇ alkanes, such as dodecane (C₁₂) and tetradecane (C₁₄), sold respectively under references PARAFOL® 12-97 and PARAFOL® 14-97 (Sasol) and as alkanes obtained according to the process described in the international application WO 2007/068371 A1, such as the mixture of undecane (C₁₁) and tridecane (C₁₃).

Among volatile hydrocarbon-based oils, isododecane is preferred.

As volatile oils, it is also possible to use volatile silicones, such as, for example, volatile linear or cyclic silicone oils, in particular those having a viscosity less than or equal to 8 centistokes (cSt) (8 × 10⁻⁶ m²/s) and in particular having from 2 to 10 silicon atoms and in particular from 2 to 7 silicon atoms, these silicones optionally comprising alkyl or alkoxyl groups having from 1 to 10 carbon atoms. Of volatile silicone which may be used in the invention, mention may be made, in particular, of dimethicones of viscosity 5 and 6 cSt, such as, for example, dimethicone sold under the name DC Toray SH200 C Fluid 5cS by Dow Corning, octamethylcyclotetrasiloxane, decamethyl (sold under the reference KF-996 by Shin Etsu), heptamethyl hexyltrisiloxane, heptamethyloctyl trisiloxane, hexa methyl disiloxane, octamethyl trisiloxane, decamethyl tetrasiloxane, dodecamethyl pentasiloxane, and mixtures thereof.

More particularly, as volatile silicone oil, mention may be made of linear or cyclic silicone oils having from 2 to 7 silicon atoms, these silicones optionally comprising alkyl or alkoxyl groups containing from 1 to 10 carbon atoms.

Preferred examples are decamethylcyclopentasiloxane (sold under the name KF-995 by Shin Etsu), dodecamethylcyclohexasiloxane (sold under the reference KF-996 by Shin Etsu) and dodecamethyl pentasiloxane.

Among the volatile silicone oils, dodecamethyl pentasiloxane is preferred. According to one embodiment, the fatty phase of the compositions of the invention comprises from 40% to 100% by weight, preferably from 60% to 98% by weight, and preferably from 80% to 95% by weight of oil relative to the total weight of the fatty phase.

### Non-volatile oils

According to one embodiment, the fatty phase of the composition present in the container of the present invention comprises at least one non-volatile oil. The fatty phase of the composition may comprise a mixture of several non-volatile oils.

By "non-volatile oil" is meant an oil remaining on the skin or keratin fiber at ambient temperature and atmospheric pressure. More specifically, a nonvolatile oil exhibits an evaporation rate strictly less than 0.01 mg/cm²/min.

The non-volatile oils may, in particular, be chosen from hydrocarbon-based, fluorinated oils and / or non-volatile silicone oils.

As nonvolatile hydrocarbon-based oil, mention may be made in particular of:
- hydrocarbon-based oils of vegetable origin, such as phytostearyl esters, such as phytostearyl oleate, physostearyl isostearate and lauroyl/octyldodecyl/ phytostearyl glutamate (AJINOMOTO, ELDEW PS203), triglycerides consisting of esters of fatty acids and of glycerol, in particular, in which the fatty acids may have chain lengths ranging from C₄ to C₃₆, and in particular C₁₈ to C₃₆, these oils may be linear or branched, saturated or unsaturated; these oils may, in particular, be heptanoic or octanoic triglycerides, shea butter oil, alfalfa, poppy oil, potimarron oil, millet oil, barley oil, quinoa oil, rye oil, chestnut oil, passionflower oil, karite, aloe oil, sweet almond oil, peach almond oil, peanut oil, argan oil, avocado oil, oil baobab, borage oil, broccoli oil, calendula oil, camelina oil, canola oil, carrot oil, safflower oil, hemp oil, rapeseed oil, cottonseed oil, coconut oil, pumpkin seed oil, wheat germ oil, jojoba oil, lily oil, oil macadamia oil, corn oil, prairie pearl oil (meadowfoam), St. John's wort oil, monoi oil, hazelnut oil, apricot kernel oil, walnut oil, olive oil, evening primrose oil, palm oil, blackcurrant seed oil, kiwi seed oil, grape seed oil, pistachio oil, potimarron oil, pumpkin oil, muscat rose oil, sesame oil, soybean oil, sunflower oil, castor oil, and oil of watermelon seeds, and mixtures thereof, or caprylic acid/ capric triglycerides, such as those sold by the company STEARINERIES Dubois or those sold under the names Miglyol 810®, 812® and 818® by the company Dynamit Nobel;
- synthetic esters, such as oils of formula R₁COOR₂, in which R₁ represents a radical of a linear or branched fatty acid comprising from 1 to 40 carbon atoms and R₂ represents a hydrocarbon chain, in particular a branched chain containing 1 to 40 carbon atoms with the proviso that the sum of the number of carbon atoms of the chains R₁ and R₂ is greater than or equal to 10. The esters may be chosen, for example, from alcohol and fatty acid esters, such as for example: cetostearyl octanoate, esters of isopropyl alcohol, such as isopropyl myristate, isopropyl palmitate, ethyl palmitate, 2-ethylhexyl palmitate, stearate or isostearyl isostearate, isostearyl isostearate, octyl stearate, hydroxylated esters, such as isostearyl lactate, octyl hydroxystearate, diisopropyl adipate, heptanoates, isostearyl heptanoate, octanoates, decanoates or ricino alcohols or polyalcohols, such as propylene glycol dioctanoate, cetyl octanoate, tridecyl octanoate, 4-diheptanoate and 2-ethyl ethyl palmitate, alkyl benzoate, diheptanoate of polyethylene glycol, propylene glycol diethylene 2-hexanoate, and mixtures thereof, C₁₂ to C₁₅ alcohol benzoates, hexyl laurate, neopentanoic acid esters such as isodecyl neopentanoate, neopentanoate isostearyl neopentanoate, octyldodecyl neopentanoate, isononanoic acid esters, such as isononyl isononanoate, isotridecyl isononanoate, octyl isononanoate, hydroxylated esters such as isostearyl lactate, di-isostearyl malate;
- silicone oils, such as linear or cyclic nonvolatile polydimethylsiloxanes (PDMS); polydimethylsiloxanes comprising alkyl, alkoxy or phenyl groups, during or at the end of a silicone chain, groups having from 2 to 24 carbon atoms; phenyl silicones such as phenyl trimethicones, phenyl dimethicones, phenyl trimethylsiloxy diphenyl siloxanes, diphenyl dimethicones, diphenyl methyldiphenyl trisiloxanes, 2-phenyl ethyl trimethyl siloxysilicates; and
- their mixtures.

Among the linear or branched hydrocarbons of mineral or synthetic origin, paraffin oils or petroleum jelly oil are preferably used.

Among the hydrocarbon-based oils of vegetable origin, there may preferably be mentioned vegetable oils, such as sweet almond oil, jojoba oil or macadamia oil.

Synthetic oils, such as synthetic esters, are used in particular isodecyl neopentanoate or isononyl isononanoate, and among the synthetic ethers, dicapryl ether is preferably used.

Among the non-volatile silicone oils, use is preferably made of polydimethylsiloxanes, phenyltrimethicone or alkyldimethicones such as cetyl dimethicone.

According to one embodiment, the fatty phase of the compositions of the invention does not comprise a non-volatile oil.

According to one embodiment, the fatty phase of the compositions of the invention comprises less than 60% by weight, preferably from 1% to 40% by weight and preferably from 2% to 20% by weight of nonvolatile oil (s) relative to the total weight of the fatty phase.

According to one embodiment, the fatty phase of the compositions of the invention comprises from 40% to 100%, preferably from 60% to 98%, and more particularly from 80% to 95% by weight of volatile oil (s) relative to the total weight of the fatty phase, and less than 60%, preferably from 1% to 40%, and more particularly from 2% to 20% by weight of nonvolatile oil (s) relative to the total weight of the fatty phase.

According to one embodiment, the fatty phase of the compositions of the invention represents a percentage ranging from 25% to 85%, preferably ranging from 40% to 75%, and even more preferably ranging from 50% to 70% total weight of the composition.

### Film-forming polymer

The fluid skin cosmetic composition composition advantageously comprises at least one film-forming polymer. In one embodiment the film-forming polymer comprises silicon. Preferably the film-forming polymer is a MQ resin. Suitable MQ resins include trialkylsiloxysilicates such as a trimethoxysilicate available under the trade name SR1000, a mixture of cyclopentasiloxane and trimethylsiloxysilicate available under the trade name SS4230, a mixture of dimethicone and trimethylsiloxysilicate available under the trade name SS4267, a liquid trimethylsiloxysilicate available under the trade name SR399, a diisostearoyl trimethylolpropane siloxysilicate available under the trade name SF 1318, or a phenylpropylsiloxysilicate available under the trade name Silshine151, all of which are available from GE Silicones. MQ resins including trimethyoxysilicate are also provided by Dow Corning without carrier (MQ-1600 Solid Resin), with a cyclopentasiloxane carrier (RSN-0749 Resin) or with a dimethicone carrier (593 Fluid). Most preferably the film-forming polymer is RSN-0749 Resin.

According to one embodiment, the composition present in the container of the present invention comprises the MQ resin at a concentration of 0.5% to 20%, in particular 1% to 15%, more especially from 1.5% to 10%, and preferably from 3% to 5%, by weight relative to the total weight of said composition.

In a further embodiment, the film-forming polymer is of the vinyl polymer type, having at least one unit derived from a carbosiloxane dendrimer.

The vinyl polymer has a backbone and at least one side chain, which comprises a carbosiloxane dendrimer derivative unit having a carbosiloxane dendrimer structure.

The term "carbosiloxane dendrimer structure" in the context of the present invention represents a molecular structure having branched groups having high molecular weights, said structure having a high regularity in the radial direction from the skeletal bond. Such carbosiloxane dendrimer structures are described in the form of a highly branched siloxane-silylalkylene copolymer in Japanese Patent Application Laid-Open Kokai 9-171 154.

The vinyl polymers grafted with at least one carbosiloxane dendrimer derivative unit are described in WO2012/131083 incorporated by reference.

The vinyl polymers grafted with at least one carbosiloxane dendrimer derivative unit which can be particularly suitable for the present invention are the polymers sold under the names TIB 4-100, TIB 4-101, TIB 4-120, TIB 4-130, TIB 4- 200, FA 4002 ID (TIB 4-202), TIB 4-220, FA 4001 CM (TIB 4-230) by Dow Corning.

According to one embodiment, the composition present in the container of the present invention comprises the vinyl polymer having at least one carbosiloxane dendrimer derivative unit having an active ingredient content of 0.5% to 20%, in particular 1% to 15%, more especially from 1.5% to 10%, and preferably from 3% to 5%, by weight relative to the total weight of said composition.

### Other Ingredients

The composition present in the container of the present invention may also comprise additional ingredients chosen from fillers, thickening or gelling agents, surfactants, sequestrants, perfumes, antioxidants, preservatives, UV filters or sunscreens, cosmetic active agents, such as vitamins, moisturizers, emollients, surfactants, or mixtures thereof.

In particular, the cosmetic make-up composition also comprises at least one additional ingredient chosen from fillers, thickening or gelling agents, UV-screening agents, preservatives, surfactants and mixtures thereof.

In order to achieve the required viscosity of the composition present in the container of the present invention, one or more thickening agents or gelling agents may advantageously be incorporated. A thickening or gelling agent suitable for the invention may be hydrophilic or lipophilic. As lipophilic thickeners, there may be mentioned, for example, modified clays such as modified magnesium silicate (Bentone gel VS38 of RHEOX), modified hectorites such as hectorite modified with a C₁₀ to C₂₂ fatty acid ammonium chloride, such as hectorite modified by chloride distearyl di-methyl ammonium such as, for example, that sold under the name Bentone 38V® by the company Elementis or that marketed under the name 'Bentone 38 CE "by a company RHEOX or that sold under the name "Bentone Gel VS 5V" by Elementis or that marketed under the name "Bentone Gel ISD V" by Elementis. According to one embodiment, a composition of the invention may comprise thickeners having an active ingredient content of from 0.01% to 40% by weight, in particular from 0.1% to 20% by weight, in particular from 0.3% to 15% by weight relative to the total weight of the composition. According to a preferred embodiment, the composition comprises at least one lipophilic thickener, in particular at least one modified hectorite, such as hectorite modified with a fatty acid ammonium chloride of C₁₀ to C₂₂, advantageously in a content ranging from 0.1% to 5% by weight, in particular from 0.5% to 2% by weight of active material relative to the total weight of said composition.

A composition present within the container of the present invention may also comprise at least one filler, of an organic or inorganic nature, making it possible, in particular, to give it additional complementary properties of mattness, coverage, strength and/or stability. The content of filler (s) may range from 2% to 20% by weight, in particular from 4% to 12% by weight, relative to the total weight of the said composition. The term "filler" is understood to mean the colorless or white solid particles of all shapes which are present in an insoluble and dispersed form in the medium of the composition. Of a mineral or organic nature, they make it possible to impart body or stiffness to the composition and/or softness, and uniformity to make-up. The fillers used in the compositions according to the present invention may be lamellar, globular, spherical, fibre or any other intermediate forms between these defined forms. By "spherical" is meant the shape or substantially shape of a sphere, i.e. with a sphericity index, that is to say the ratio between its largest diameter and its smallest diameter, of less than 1.2. As organic spherical fillers, mention may be made, for example, of polyamide powders and, in particular, Nylon® powders such as Nylon-1 or Polyamide 12, sold under the names ORGASOL by the company Arkema; polyethylene powders; powders of polytetrafluoroethylene (Teflon *); microspheres based on acrylic copolymers, such as those of ethylene glycol dimethacrylate/lauryl methacrylate copolymer marketed by the company Dow Corning under the name POLYTRAP; expanded powders such as hollow microspheres and in particular microspheres sold under the name EXPANCEL by the company Kemanord Plast or under the name MICROPEARL F 80 ED by the company Matsumoto; silicone resin microbeads such as those sold under the name TOSPEARL by the company Toshiba Silicone; organopolysiloxane elastomer powders, preferably non-emulsifying, such as those marketed under the name KSP 100 by Shin Etsu (INCI name: vinyl dimethicone/methicone silsesquioxane crosspolymer); the microspheres of polymethyl methacrylate marketed under the name MICROSPHERE M-100 by the company Matsumoto or under the name COVABEAD LH85 by the company Wackherr; ethylene-acrylate copolymer powders, such as those marketed under the name FLOBEADS by the company Sumitomo Seika Chemicals; powders of natural organic materials such as starch powders, in particular corn starch, wheat or rice, crosslinked or not, such as starch powders crosslinked with octenylsuccinate anhydride, marketed under the name DRY-FLO by the company National Starch; metal soaps derived from organic carboxylic acids having from 8 to 22 carbon atoms, preferably from 12 to 18 carbon atoms, for example, zinc, magnesium or lithium stearate, zinc laurate, myristate magnesium, Polypore * L 200 (Chemdal Corporation), polyurethane powders, in particular crosslinked polyurethane powders comprising a copolymer, said copolymer comprising trimethylol hexyllactone, such as hexamethylene diisocyanate/trimethylol hexyllactone polymer, sold under the name PLASTIC POWDER D-400® or PLASTIC POWDER D-800® by TOSHIKI, microcircles from Carnauba, such as that marketed under the name MicroCare 350® by the company MICRO POWDERS, synthetic wax microcircles , such as that marketed under the name of MicroEase 1 14S® by the company MICRO POWDERS, microcircles consisting of a mixture of wax Carnauba wax and polyethylene wax, such as those marketed under the names of Micro Care 300® and 310® by the company Micro Powders, microcircles consisting of a mixture of Carnauba wax and synthetic wax, such as that marketed under the name Micro Care 325® by MICRO POWDERS, polyethylene microcircles, such as those marketed under the names Micropoly 200®, 220®, 220L® and 250S® by the company MICRO POWDERS. As mineral spherical filler, mention may especially be made of airgel particles, preferably silylated silica airgel particles (INCI name silica silylate), and more particularly particles of surface-modified hydrophobic silica airgel particles with trimethylsilyl groups (trimethylsiloxylated silica), and poly (metal oxides). The term "lamellar" means parallelepipedal (rectangular or square surface), discoidal (circular surface) or ellipsoidal (oval surface), characterized by three dimensions: length, width and height. As lamellar fillers which can be used in the present invention, mention may be made, for example, of talcs, micas, perlites or mixtures thereof.

The composition present within the container of the present invention may also comprise at least one ultraviolet (UV) filter, preferably an insoluble and/or lipophilic organic (s) UV filter(s). The term "insoluble UV filter" is understood to mean any UV filter capable of being in the form of particles in a liquid fatty phase and in a liquid aqueous phase, such as insoluble organic UV screening agents and inorganic filters. As an insoluble UV filter, mention may be made, for example, of metal (poly) oxides. The term "lipophilic organic UV screen" is understood to mean an organic molecule capable of filtering UV radiation between 290 and 400 nm and which can be solubilized in molecular state or dispersed in an oily phase in order to obtain a macroscopically homogeneous phase. As the lipophilic organic filter, mention may be made in particular of cinnamic derivatives; anthranilates; salicylic derivatives, dibenzoylmethane derivatives, camphor derivatives; benzophenone derivatives; β,β-diphenylacrylate derivatives; triazine derivatives; benzotriazole derivatives; phenyl benzotriazole derivatives; benzalmalonate derivatives; imidazolines; p-aminobenzoic acid derivatives (PABA); benzoxazole derivatives; filter polymers and silicone filters; dimers derived from oalkylstyrene; 4,4-diarylbutadienes; merocyanine derivatives; and mixtures thereof. In a preferred embodiment, the lipophilic organic filter may be chosen from ethylhexyl methoxycinnamate sold in particular under the trade name "Parsol MCX" by DSM Nutritional Products. According to one embodiment, the UV filter(s), preferably an insoluble and/or lipophilic organic UV filter(s), is (are) present in the composition present within the container of the present invention in a content ranging from 0.1 to 50% by weight, relative to the total weight of the composition, preferably in a content ranging from 1 to 40% by weight, in particular ranging from 3 to 25% by weight, relative to the total weight of the composition.

The composition present within the container of the present invention may also comprise at least one surfactant or emulsifier. In this regard, a wide variety of silicone emulsifiers are useful herein. These silicone emulsifiers are typically organically modified organopolysiloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and chains comprising moieties derived from both ethylene oxide and propylene oxide. Other examples include alkyl-modified dimethicone copolyols, in other words compounds which comprise C2-C30 pendant side chains. Still other useful dimethicone copolyols include materials having various cationic, anionic, amphoteric and zwitterionic pendant moieties.

The emulsifiers and/or surfactants may be in particular anionic or nonionic, and may be hydrocarbon or silicone based. The emulsifiers and/or surfactants may have an HLB (hydrophilic-lipophilic balance) balance at 25 °C in the sense of GRIFFIN, greater than or equal to 8 or less than 8, or a mixture thereof. The HLB value according to GRIFFIN is defined in J. Soc. Cosm. Chem. 1954 (vol. 5), pp. 249-256. Reference can be made to the document "Encyclopedia of Chemical Technology, Kirk-Othmer", Vol. 22, p. 333-432, 3rd edition, 1979, Wiley, for the definition of the emulsifying properties and functions of the surfactants, in particular p. 347-377 of this reference. The composition present in the container of the present invention may comprise at least one silicone nonionic surfactant chosen from polydimethyl (or dialkyl) silicones with polyoxyalkylenated (polyoxyethylenated (or POE) and/or polyoxypropylenated (or PPO) hydrophilic side- and / or terminal groups and / or end groups containing C₁ to C₂₀ alkyl side groups, polydialkyl silicones with polyglycerolated or glycerolated side and/or terminal groups and mixtures thereof. The silicone nonionic surfactant is preferably chosen from:
- Polydimethyl (or dialkyl) silicones with polyoxyethylene (or POE) and/or polyoxypropylenated (or PPO) polyoxyalkylenated hydrophilic and/or polyoxyalkylenated end groups and groups, having more hydrophobic alkyl side groups than the above-mentioned side and/or terminal groups C₁ to C₂₀, and preferably C₄ to C₂₀, linear or branched, preferably linear alkyl groups, such as lauryl or cetyl. These surfactants can also carry organosiloxane side groups;
- polydimethyl siloxanes with POE side groups and alkyl side groups, such as cetyl PEG-PPG 10/1 dimethicone, sold under the name ABIL EM90 by the company Evonik GOLDSCHMIDT;
- branched polydimethyl siloxanes with alkyl side groups, such as, in particular, lauryl PEG-9 polydimethylsiloxyethyl dimethicone, marketed under the name KF-6038 by the company Shin Etsu;
- Polydialkyl silicones with polyglycerolated or glycerolated side and / or terminal groups. These silicone surfactants preferably additionally contain linear or branched C₁ to C₂₀ alkyl side groups, and preferably also linear alkyl groups such as lauryl or cetyl. Likewise, these silicone and glycerolated surfactants can additionally carry organosiloxane side groups;
- polydimethyl siloxanes with polyglycerolated side groups, such as polyglyceryl-3-disiloxane dimethicone, marketed under the name KF-6100 by the company Shin Etsu;
- polydimethyl siloxanes branched and with polyglycerolated side groups, such as polyglyceryl-3 polydimethylsiloxyethyl dimethicone, marketed under the name KF-6104 by the company Shin Etsu;
- branched polydimethylsiloxanes with polyglycerolated side groups and alkyl side groups, such as lauryl polyglyceryl-3 polydimethyl siloxyethyl dimethicone, marketed under the name KF-6105 by the company Shin Etsu;
- dimethicone copolyol, such as that sold under the name Q2-5220® by the company Dow Corning;
- dimethicone copolyol benzoate (FINSOLV SLB 101® and 201® from FINTEX);
- the mixture of cyclomethicone / dimethicone copolyol sold under the name Q2-3225C® by the company Dow Corning;
- polydimethyl siloxanes with POE side and / or terminal groups, such as, in particular, KF-601 1, KF-6012, KF-6013, KF-6015, KF-6016 and KF-6017 from Shin Etsu;
- bis-PEG/PPG-14/14 dimethicone & cyclopentasiloxane sold under the name ABIL EM97 by the company Evonik GOLDSCHMIDT;
- branched polydimethylsiloxanes with POE side groups, such as in particular PEG-9 polydimethyl siloxyethyl dimethicone, sold under the name KF-6028 by the company Shin Etsu; and
- mixtures thereof.

According to a particular embodiment, the non-ionic silicone surfactant may be a C₈ to C₂₂ alkyl dimethicone copolyol, that is to say a polyethyl (C₈ to C₂₂) dimethyl methyl siloxane oxypropylenated and/or oxyethylenated, in particular the product marketed under the name Abil EM-90 by the company Goldschmidt. The composition present in the container of the present invention may comprise at least one nonionic hydrocarbon surfactant. Non-ionic hydrocarbon surfactants which may be mentioned include fatty acid esters of polyols such as sorbitol or glycerol mono-, di-, tri- or sesqui-oleates or stearates, glycerol or polyethylene glycol laurates; fatty acid esters of polyethylene glycol (polyethylene glycol monostearate or monolaurate); polyoxyethylenated sorbitol fatty acid esters (stearate, oleate); alkyl (lauryl, cetyl, stearyl, octyl) polyoxyethylene ethers. Among the nonionic hydrocarbon surfactants, the following are preferred:
- polyglycerolated fatty acid esters comprising at least 3 glycerol ether units, such as polyglyceryl 3;
- polyoxyalkylenated (polyoxyethylenated and/or polyoxypropylenated) fatty acid esters, preferably comprising at least 3 oxyethylene groups;
- ethers of fatty alcohols and polyglycerols with at least 3 glyceryl ether units;
- polyoxyalkylene fatty alcohol ethers (POE and/or POE/PPO) with at least 3 POE groups;
- esters and ethers of bones such as the mixture of cetylstearyl glucoside and cetyl and stearyl alcohols, such as Montanov 68 from Seppic;
- oxyethylenated and/or oxypropylenated ethers (which may contain from 1 to 150 oxyethylene and/or oxypropylenated groups) of glycerol;
- oxyethylenated and/or oxypropylenated ethers (which may comprise from 1 to 150 oxyethylene and/or oxypropylenated groups) of fatty alcohols (in particular of C₈ to C₂₄ and preferably C₁₂ to C₁₈ alcohol), such as oxyethylenated ether cetearyl alcohol with 30 oxyethylene groups (CTFA name "Ceteareth-30"), oxyethylene ether of stearyl alcohol with 20 oxyethylene groups (CTFA name "Steareth-20"), and the oxyethylene ether of the mixture of C₁₂ to C₁₅ fatty alcohols comprising 7 oxyethylenated groups (CTFA name "C₁₂₋₁₅ Pareth-7") marketed under the name NEODOL 25-7® by SHELL CHEMICALS;
- fatty acid esters (in particular C₈ - C₂₄ and preferably C₁₆ - C₂₂ acids) and polyethylene glycol (which may comprise from 1 to 150 ethylene glycol units), such as PEG-50 stearate and the monostearate of PEG-40 marketed under the name MYRJ 52P® by the company ICI UNIQUEMA;
- fatty acid esters (especially C₈ to C₂₄ and preferably C₁₆ to C₂₂ acids) and oxyethylenated and/or oxypropylenated glycerol ethers (which may comprise from 1 to 150 oxyethylene and/or oxypropylenated groups), such as the monostearate of PEG-200 glyceryl sold under the name Simulsol 220 TM® by the company SEPPIC; polyethoxylated glyceryl stearate containing 30 ethylene oxide groups, such as the product TAGAT S® sold by GOLDSCHMIDT, polyethoxylated glyceryl oleate containing 30 ethylene oxide groups, such as the product TAGAT O® sold by the group the GOLDSCHMIDT company, polyethoxylated glyceryl cocoate containing 30 ethylene oxide groups, such as the product VARIONIC L1 13® sold by the company SHEREX, glyceryl isostearate polyethoxylated with 30 ethylene oxide groups, such as the product TAGAT L® sold by GOLDSCHMIDT and polyethoxylated glyceryl laurate containing 30 ethylene oxide groups, such as TAGAT I® from GOLDSCHMIDT;
- fatty acid esters (in particular C₈ to C₂₄ preferably C₁₆ to C₂₂ acids) and oxyethylenated and/or oxypropylenated (possibly containing from 1 to 150 oxyethylene and/or oxypropylenated) oxyethylenated and/or oxypropylenated sorbitol ethers; such as the polysorbate sold under the name Tween 20® by the company CRODA, the polysorbate 60 sold under the name Tween 60® by the company CRODA;
- copolymers of propylene oxide and of ethylene oxide, also called EO/PO polycondensates;
- esters and ethers of bones such as sucrose stearate, sucrose cocoate, sorbitan stearate and mixtures thereof, such as Arlatone 2121® sold by the company ICI;
- oxyethylenated and/or oxypropylenated ethers (which may comprise from 1 to 150 oxyethylene and/or oxypropylenated groups) of fatty alcohols (in particular of C₈ to C₂₄ and preferably C₁₂ to C₁₈ alcohol), such as oxyethylenated ether stearyl alcohol containing 2 oxyethylene groups (CTFA name "Steareth-2");
- fatty acid esters of fatty acids (in particular C₈ to C₂₄ and preferably C₁₆ to C₂₂ acids) and of polyol, in particular of glycerol or sorbitol, such as glyceryl stearate, glyceryl stearate such as the product sold under the name TEGIN M® by the company GOLDSCHMIDT, glyceryl laurate, such as the product sold under the name IMWITOR 312® by the company HULS, 2-polyglyceryl stearate, sorbitan tristearate, glyceryl ricinoleate;
- lecithins, such as soya lecithins (such as Emulmetik 100 J from Cargill, or Biophilic H from Lucas Meyer); and
- mixtures thereof.

Among the nonionic hydrocarbon surfactants, preferred are polyglyceryl-4 isostearate sold under the name Isolan GI 34® by the company Evonik Goldschmidt.

The anionic surfactant(s) which can be used in the context of the present invention is (are) in particular (non-limiting list) one or more salts (in particular alkali salts, and especially sodium salts, ammonium salts, amine salts, aminoalcohol salts or magnesium salts) of the following compounds:
- alkylphosphates, and in particular C₁₂ to C₂₄, preferably C₁₄ to C₁₈ alkyl phosphates and mixtures thereof, in particular they may be chosen from DEA oleth-10 phosphate (Crodafos N 10N from the company CRODA), cetyl phosphate (Amphisol K from Givaudan or ARLATONE MAP 160K from UNIQEMA), stearyl phosphate and cetearyl phosphate;
- alkylsulfates, alkyl ether sulfates (such as sodium lauryl ether sulfate), alkylamido ether sulfates, alkylaryl polyether sulfates, monoglycerides sulfates;
- alkylsulfonates, alkylamide sulfonates, alkylarylsulfonates, D-olefin sulfonates, paraffin sulfonates;
- alkylsulfosuccinates, alkyl ether sulfosuccinates, alkylamidesulfosuccinates, such as "Disodium PEG-5 citrate lauryl sulfosuccinate" and "Disodium ricinoleamido MEA sulfosuccinate";
- alkylsulfosuccinamates;
- alkylsulfoacetates;
- acylsarcosinates, acylglutamates such as "Disodium hydrogenated tallow glutamate" (AMISOFT HS-21 R marketed by Aminomoto), acylisethionates, N-acyltaurates and acyl lactylates;
- alkyl (poly) glycoside carboxylic esters such as alkylglucoside citrates, alkylpolyglycoside tartrate and alkylpolyglycoside sulfosuccinates, alkylpolyglycoside sulfosuccinamates;
- fatty acids, in particular fatty acid salts and in particular amine salts or alkali metal salts, such as the salts of oleic, ricinoleic, palmitic, stearic acids, coconut oil or oil acids hydrogenated copra;
- alkyl galactoside uronic acids and their salts, polyoxyalkylenated alkyl ether carboxylic acids, polyoxyalkylenated alkyl aryl ether carboxylic acids, polyoxyalkylenated alkyl amido ether carboxylic acids and their salts, in particular those comprising from 2 to 50 oxide groups alkylene, in particular ethylene; and
- mixtures thereof.

The anionic surfactant preferably comprises at least one cationic counterion.

This cationic counterion may be of mineral origin, in particular chosen from alkali metal and alkaline-earth metal cations, or organic cations.

The alkali metal cation(s) may be selected from sodium and potassium.

The alkaline earth metal cation(s) may be selected from calcium and magnesium.

The cation(s) of organic origin may be chosen from ammonium, its amine derivatives and aminoalcohols, or magnesium. Preferably, the cation(s) of organic origin is chosen from ammonium, and its amine derivatives and aminoalcohols.

According to a preferred embodiment, the cation is a primary (poly) hydroxyalkylamine.

The term primary (poly) hydroxyalkylamine is understood to mean in particular a primary dihydroxyalkylamine, it being understood that by primary is meant a primary amine function, i.e. -NH₂, and the alkyl group being a linear or branched C₁ to C₈ hydrocarbon chain, preferably a branched C₄, such as 1,3-dihydroxy-2-methylpropyl.

The primary poly (hydroxy) alkylamine is preferably 1,3-dihydroxy-2-methyl-2-propylamine (also known as aminomethyl propanediol or AMPD).

Each of these nonionic and anionic surfactants can be used alone or as a mixture with one or more other anionic and/or nonionic surfactants.

According to one embodiment, the surfactant(s) and/or emulsifier(s) is (are) present in the composition present in the container of the present invention in a content ranging from 0.1 to 15% by weight, preferably a content ranging from 1 to 10% by weight, relative to the total weight of the composition.

Of course, those skilled in the art will be careful to select any complementary compounds and/or their quantity in such a way that the advantageous properties intrinsically attached to the composition present in the container of the present invention and its packaging and distribution with the device according to the invention do not are not, or substantially not, altered by the proposed addition.

Unless otherwise stated, the values in the above examples are expressed as % by weight based on the total weight of the composition.

### Methods

In a further aspect, the present invention provides a method of converting a skincare composition into a colour cosmetic composition comprising dispensing one to five drop(s), preferably one to three drops, from the container of the present invention onto an amount of skincare composition suitable for application to one or more of the skin regions selected from the list consisting of (a) one or more specific parts of the face; (b) the face as a whole; (c) the neck; and (d) the décolletage.

In a further aspect, the present invention provides a method of altering the colour of a colour cosmetic composition comprising dispensing one to five drop(s), preferably one to three drop(s), from the container of the present invention onto an amount of colour cosmetic composition suitable for application to one or more of the skin regions selected from the list consisting of (a) one or more specific parts of the face; (b) the face as a whole; (c) the neck; and (d) the décolletage.

With respect to these methods, the skincare composition is a composition that does not comprise any coloured (non-white) pigments. Thus skincare composition are generally white or clear in appearance. Such skincare compositions may be used to moisturise the skin, to treat or prevent blemishes such as acne, to improve skin health or to reduce the signs of ageing. Such skincare compositions include sunscreen formulations, serums, moisturisers (including emollients such as lotions, sprays, creams and ointments), oils and face masks.

By contrast, the colour cosmetic composition discussed above (i.e. the colour cosmetic composition not present in the container) is a formulation that comprises coloured pigments. These compositions may comprise skincare actives as well. Such colour cosmetic compositions include foundations, concealers, BB creams, CC creams, DD creams, blushers, bronzers, highlighters and primers.

The number of drops dispensed will vary based on the level of coverage the consumer desires, whether the drops are dispensed onto a skincare composition or a colour cosmetic composition (generally more drops will be dispensed onto a skincare composition) and the quantity of skincare composition or of colour cosmetic composition the drops are being applied to.

The skilled person will understand that optional features of one embodiment or aspect of the invention may be applicable, where appropriate, to other embodiments or aspects of the invention.

There now follows by way of example only a description of the present invention with reference to the accompanying drawings, in which:
**Figure 1** shows a longitudinal section of an example container of the present invention; and
**Figure 2** shows the weight of high-pigment formulation droplets when dispensed from a tottle with a dispensing head comprising a circular dispensing orifice of 0.785 mm² containing either Formulation 1 (according to the present invention) or a benchmark formulation. The bars represent the range from the mean minus standard deviation to the mean plus standard deviation, with the middle line representing the mean.

### EXAMPLES

### Example 1 - Viscosity determination

The viscosity of Formulation 1 (comprising Aqua (Water), Caprylyl methicone, Caprylic/capric triglyceride, PEG-9 dimethicone, Bis-isobutyl PEG/PPG-10/7dimethicone copolymer, Stearalkonium hectorite, Phenoxyethanol, Magnesium sulfate, Aluminum hydroxide, Triethoxycaprylylsilane, Propylene carbonate, Xanthan gum, Methylparaben, PEG/PPG dimethylallylether, Ethylparaben, Tetrasodium EDTA, Tocopherol, CI 77891 (Titanium dioxide), CI 77492 (Iron oxides), CI 77491 (Iron oxides) and CI 77499 (Iron oxides), with a total pigment concentration of 28%) and a benchmark formulation (comprising an ingredient list equivalent to that of Formulation 1 and with a total pigment concentration of 25%) was determined. Viscosity as described in the present application is measured using a Brookfield LVDV-I Prime E viscometer plus Model G Laboratory Stand, equipped with LV Spindle 64. Viscosity measurements were obtained as follows:
1. Ensure the sample product has a temperature of 23°C and that it is not aerated. Sample is presented in a 60ml capacity plastic container with a diameter of 35mm and a height of 70mm;
2. Before measurement, auto-zero the viscometer after switching on the unit by following the on-screen instructions with no spindle attached to the viscometer;
3. Select the spindle 64;
4. Select the revolution speed "12". This will rotate the spindle at 12 revolutions per minute (rpm);
5. Carefully attach the spindle to the lower shaft of the viscometer;
6. Lower the spindle into the sample product until the fluid level is at the immersion groove on the spindle shaft by turning the height adjustment knob on the right hand side of the viscometer mounting bracket. Ensure that the spindle is not in contact with the sides or base of the sample container;
7. Press the "Timed Option" buttons;
8. Use the Up and Down arrows to select the "Timed Stop" option then press "Enter" to confirm;
9. Use the Up and Down arrows to select zero minutes, then press "Enter" to confirm;
10. Use the Up and Down arrows to select 30 seconds , then press "Enter" to confirm;
11. Press the "Motor On/Off" button to begin the measurement;
12. The viscometer will display a countdown from 30 seconds, after which it will display the final viscosity measured; and
13. Record the viscosity reading.

Formulation 1 has a viscosity of 26,000 cps. By contrast, the benchmark formulation has a viscosity of 1,250 cps. Thus, Formulation 1 has a much high viscosity that falls within the scope of the present invention, whilst the benchmark formulation does not.

### Example 2 - Variation in droplet weight

Formulation 1 and the benchmark formulation were sealed within a tottle with a dispensing head comprising a circular dispensing orifice of 0.785 mm².

Twenty droplets of formulation 1 and the benchmark formulation were expelled whilst the tottle was held in a substantially vertical, dispensing-head-down position. The weight of each of these droplets is presented in Table 1 below.

**Table 1**

| | Formulation 1 | Benchmark |
|---|---|---|
| | 0.0334 | 0.0216 |
| | 0.0316 | 0.0322 |
| | 0.0352 | 0.0301 |
| | 0.0343 | 0.0301 |
| | 0.0307 | 0.0238 |
| | 0.0383 | 0.0259 |
| | 0.0273 | 0.0254 |
| | 0.038 | 0.0258 |
| | 0.0361 | 0.0163 |
| | 0.0361 | 0.0307 |
| | 0.0341 | 0.0277 |
| | 0.0303 | 0.0335 |
| | 0.0365 | 0.0291 |
| | 0.0325 | 0.0285 |
| | 0.0328 | 0.0326 |
| | 0.0326 | 0.0267 |
| | 0.0353 | 0.0296 |
| | 0.0329 | 0.0273 |
| | 0.0396 | 0.0250 |
| | 0.0303 | 0.0194 |
| Mean | **0.0339** | **0.0271** |
| Standard Deviation | **0.0030** | **0.0043** |

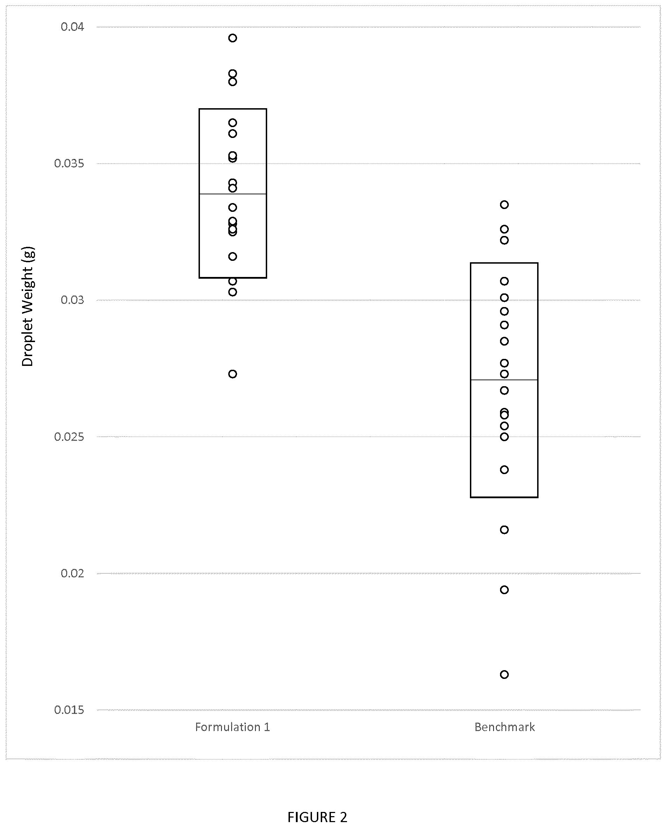

Table 1 and Figure 2 show that a much more consistent droplet size is seen when a formulation according to the present invention is used. In particular, a decrease in the droplet weight standard deviation by more than a quarter is seen in Formulation 1 when compared to the Benchmark formulation. The increased variation of droplet size seen in the benchmark composition is a result of the tottle of the benchmark composition having an increased tendency to sporadically expel an unusually small quantity of formulation in one droplet. The sporadic release of such small droplets is undesirable as it is likely to be much less than a user needs.

## Claims

1. A container (1) comprising a flexible wall (2) and a dispensing head (3), wherein:
(i) the dispensing head (3) is not detachable from the rest of the container (1) during normal use;
(ii) the dispensing head (3) comprises a dispensing orifice (7);
(iii) the smallest cross-sectional area of passage through the dispensing orifice (X) is between 0.2 mm² and 2.0 mm²; and
(iv) the container (1) contains a fluid skin colour cosmetic composition comprising at least 20% of one or more pigments;
**characterised in that** the fluid skin colour cosmetic composition has a viscosity of at least 10 Pa.s at 23 °C.

2. The container (1) of claim 1, wherein the fluid skin cosmetic composition has a viscosity of less than 70 Pa.s measured at 23 °C.

3. The container (1) of claim 1 or claim 2, wherein the fluid skin cosmetic composition has a viscosity of at least 20 Pa.s, preferably at least 22 Pa.s, more preferably at least 23 Pa.s, more preferably at least 24 Pa.s, more preferably at least 25 Pa.s, measured at 23 °C.

4. The container (1) of any one of claims 1 to 3, wherein the fluid skin cosmetic composition comprises no more than 40% of one or more pigments.

5. The container (1) of any one of claims 1 to 4, wherein the fluid skin cosmetic composition comprises at least 22% of one or more pigments, preferably at least 24% of one or more pigments, more preferably at least 26% of one or more pigments, more preferably at least 27% of one or more pigments.

6. The container (1) of any one of claims 1 to 5, wherein the container (1) is a bottle, preferably a tottle.

7. The container (1) of any one of claims 1 to 6, wherein the smallest cross-sectional area of passage through the dispensing orifice (X) is between 0.3 mm² and 2.0 mm², preferably between 0.3 mm² and 1.6 mm², more preferably between 0.4 mm² and 1.2 mm², more preferably between 0.6 mm² and 1.0 mm².

8. The container (1) of any one of claims 1 to 7, wherein the one or more pigments present in the fluid skin cosmetic composition comprise inorganic pigments.

9. The container (1) of claim 8, wherein the one or more pigments comprise titanium dioxides, iron oxides, ultramarines, chromium oxides, chromium hydroxides or mixtures thereof.

10. The container (1) of any one of claims 1 to 9, wherein the fluid skin cosmetic composition is either a pigment drop composition or a concealer composition.

11. A method of converting a skincare composition into a colour cosmetic composition or of altering the colour of a colour cosmetic composition comprising dispensing one to five drop(s) from the container (1) as defined in any one of claims 1 to 10 onto an amount of skincare composition or of the colour cosmetic composition suitable for application to one or more of the skin regions selected from the list consisting of (a) one or more specific parts of the face; (b) the face as a whole; (c) the neck; and (d) the décolletage.

## Patentansprüche

1. Behälter (1), umfassend eine flexible Wand (2) und einen Dosierkopf (3), wobei:
(i) der Dosierkopf (3) während eines normalen Gebrauchs nicht vom Rest des Behälters (1) abnehmbar ist;
(ii) der Dosierkopf (3) eine Dosieröffnung (7) aufweist;
(iii) die kleinste Querschnittsfläche eines Durchgangs durch die Dosieröffnung (X) zwischen 0,2 mm² und 2,0 mm² ist; und
(iv) der Behälter (1) eine hautfarbene kosmetische Fluidzusammensetzung, umfassend mindestens 20 % eines oder mehrerer Pigmente, enthält;
**dadurch gekennzeichnet**, das die hautfarbene kosmetische Fluidzusammensetzung eine Viskosität von mindestens 10 Pa.s bei 23 °C aufweist.

2. Behälter (1) nach Anspruch 1, wobei die hautkosmetische Fluidzusammensetzung eine Viskosität von weniger als 70 Pa.s, gemessen bei 23 °C, aufweist.

3. Behälter (1) nach Anspruch 1 oder Anspruch 2, wobei die hautkosmetische Fluidzusammensetzung eine Viskosität von mindestens 20 Pa.s, bevorzugt mindestens 22 Pa.s, stärker bevorzugt mindestens 23 Pa.s, stärker bevorzugt mindestens 24 Pa.s, stärker bevorzugt mindestens 25 Pa.s aufweist, gemessen bei 23 °C.

4. Behälter (1) nach einem der Ansprüche 1 bis 3, wobei die hautkosmetische Fluidzusammensetzung nicht mehr als 40 % eines oder mehrerer Pigmente umfasst.

5. Behälter (1) nach einem der Ansprüche 1 bis 4, wobei die hautkosmetische Fluidzusammensetzung mindestens 22 % eines oder mehrerer Pigmente, bevorzugt mindestens 24 % eines oder mehrerer Pigmente, stärker bevorzugt mindestens 26 % eines oder mehrerer Pigmente, stärker bevorzugt mindestens 27 % eines oder mehrerer Pigmente umfasst.

6. Behälter (1) nach einem der Ansprüche 1 bis 5, wobei der Behälter (1) eine Flasche, vorzugsweise eine Tottle ist.

7. Behälter (1) nach einem der Ansprüche 1 bis 6, wobei die kleinste Querschnittsfläche eines Durchgangs durch die Dosieröffnung (X) zwischen 0,3 mm² und 2,0 mm², vorzugsweise zwischen 0,3 mm² und 1,6 mm², stärker bevorzugt zwischen 0,4 mm² und 1,2 mm², stärker bevorzugt zwischen 0,6 mm² und 1,0 mm² ist.

8. Behälter (1) nach einem der Ansprüche 1 bis 7, wobei das eine oder die mehreren Pigmente, die in der hautkosmetischen Fluidzusammensetzung vorhanden sind, anorganische Pigmente umfassen.

9. Behälter (1) nach Anspruch 8, wobei das eine oder die mehreren Pigmente Titandioxide, Eisenoxide, Ultramarine, Chromoxide, Chromhydroxide oder Gemische davon umfassen.

10. Behälter (1) nach einem der Ansprüche 1 bis 9, wobei die hautkosmetische Fluidzusammensetzung entweder eine Pigment-Drop-Zusammensetzung oder eine Concealer-Zusammensetzung ist.

11. Verfahren zum Umwandeln einer Hautpflegezusammensetzung in eine kosmetische Farbzusammensetzung oder zum Ändern der Farbe einer kosmetischen Farbzusammensetzung, umfassend Dosieren von einem bis fünf Tropfen aus dem Behälter (1), wie in einem der Ansprüche 1 bis 10 definiert, auf eine Menge einer Hautpflegezusammensetzung oder der kosmetischen Farbzusammensetzung, die zum Auftragen auf eine oder mehrere Hautregionen, ausgewählt aus der Liste bestehend aus (a) einem oder mehreren spezifischen Teilen des Gesichts; (b) dem Gesicht als Ganzes; (c) dem Hals; und (d) dem Dekolleté, geeignet ist.

## Revendications

1. Récipient (1) comprenant une paroi flexible (2) et une tête de distribution (3), dans lequel :
(i) la tête de distribution (3) n'est pas détachable du reste du récipient (1) pendant une utilisation normale ;
(ii) la tête de distribution (3) comprend un orifice de distribution (7) ;
(iii) la plus petite surface en coupe transversale du passage à travers l'orifice de distribution (X) est comprise entre 0,2 mm² et 2,0 mm² ; et
(iv) le récipient (1) contient une composition cosmétique fluide de couleur peau comprenant au moins 20 % d'un ou plusieurs pigments ;
**caractérisé en ce que** la composition cosmétique fluide de couleur peau a une viscosité d'au moins 10 Pa.s à 23°C.

2. Récipient (1) selon la revendication 1, dans lequel la composition cosmétique fluide pour la peau a une viscosité, mesurée à 23°C, inférieure à 70 Pa.s.

3. Récipient (1) selon la revendication 1 ou la revendication 2, dans lequel la composition cosmétique fluide pour la peau a une viscosité, mesurée à 23°C, d'au moins 20 Pa.s, de préférence d'au moins 22 Pa.s, mieux encore d'au moins 23 Pa.s, plus particulièrement d'au moins 24 Pa.s, tout spécialement d'au moins 25 Pa.s.

4. Récipient (1) selon l'une quelconque des revendications 1 à 3, dans lequel la composition cosmétique fluide pour la peau comprend au plus 40 % d'un ou plusieurs pigments.

5. Récipient (1) selon l'une quelconque des revendications 1 à 4, dans lequel la composition cosmétique fluide pour la peau comprend au moins 22 % d'un ou plusieurs pigments, de préférence au moins 24 % d'un ou plusieurs pigments, mieux encore au moins 26 % d'un ou plusieurs pigments, plus particulièrement au moins 27 % d'un ou plusieurs pigments.

6. Récipient (1) selon l'une quelconque des revendications 1 à 5, lequel récipient (1) est une bouteille, de préférence une bouteille au format tête en bas.

7. Récipient (1) selon l'une quelconque des revendications 1 à 6, dans lequel la plus petite surface en coupe transversale du passage à travers l'orifice de distribution (X) est comprise entre 0,3 mm² et 2,0 mm², de préférence entre 0,3 mm² et 1,6 mm², mieux encore entre 0,4 mm² et 1,2 mm², plus particulièrement entre 0,6 mm² et 1,0 mm².

8. Récipient (1) selon l'une quelconque des revendications 1 à 7, dans laquelle le ou les pigments présents dans la composition cosmétique fluide pour la peau comprennent des pigments inorganiques.

9. Récipient (1) selon la revendication 8, dans lequel le ou les pigments comprennent des dioxydes de titane, des oxydes de fer, des outremers, des oxydes de chrome, des hydroxydes de chrome ou leurs mélanges.

10. Récipient (1) selon l'une quelconque des revendications 1 à 9, dans lequel la composition cosmétique fluide pour la peau est soit une composition de gouttes de pigment soit une composition de fond de teint.

11. Procédé pour convertir une composition de soin de la peau en une composition cosmétique de couleur ou pour altérer la couleur d'une composition cosmétique de couleur, comprenant la distribution d'une à cinq gouttes à partir du récipient (1) tel que défini dans l'une quelconque des revendications 1 à 10 sur une quantité de composition de soin de la peau appropriée pour une application sur une ou plusieurs des régions cutanées choisies dans la liste comprenant (a) une ou plusieurs parties spécifiques du visage ; (b) le visage dans son ensemble ; (c) le cou ; et (d) le décolleté.
